(19) Europäisches Patentamt
European Patent Office
Office européen des brevets

(11) Publication number: 0 132 501
A2

# (12) EUROPEAN PATENT APPLICATION

(21) Application number: 84103435.8

(22) Date of filing: 28.03.84

(51) Int. Cl.[4]: C 07 D 501/36
A 61 K 31/545

(30) Priority: 31.03.83 JP 56895/83
08.07.83 JP 124155/83

(43) Date of publication of application:
13.02.85 Bulletin 85/7

(84) Designated Contracting States:
CH DE FR GB LI

(71) Applicant: AJINOMOTO CO., INC.
5-8, Kyobashi 1-chome, Chuo-ku
Tokyo 104(JP)

(72) Inventor: Yasuda, Naohiko
No. 2-36-2, Hairando
Yokosuka-shi Kanagawa-ken(JP)

(72) Inventor: Nakanishi, Eiji
No. 2-20-8, Kannon Kawasaki-ku
Kawasaki-shi Kanagawa-ken(JP)

(72) Inventor: Ogasawara, Yoshiaki
No. 2-20-8, Kannon Kawasaki-ku
Kawasaki-shi Kanagawa-ken(JP)

(72) Inventor: Kato, Toshihisa
No. 2-20-8, Kannon Kawasaki-ku
Kawasaki-shi Kanagawa-ken(JP)

(72) Inventor: Sasaki, Yukio
No. 2-20-8, Kannon Kawasaki-ku
Kawasaki-shi Kanagawa-ken(JP)

(74) Representative: Schübel-Hopf, Ursula et al,
Strehl, Schübel-Hopf, Schulz Patentanwälte
Widenmayerstrasse 17
D-8000 München 22(DE)

## (54) Cephalosporin derivatives, their preparation and pharmaceutical compositions.

(57) It has been found that imidazole dicarboxylic acid derivatives of the general formula

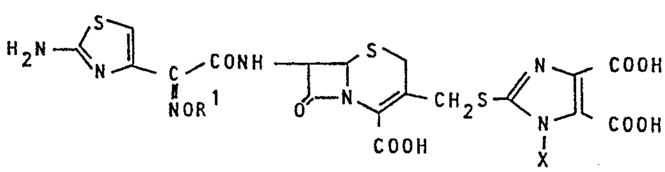

wherein R' represents a lower alkyl group and X represents a hydrogen atom or a lower alkyl group which optionally has a substituent, and in which the amino group may carry a protective group, esters and salts thereof, show high activity against a broad range of gram-positive and gram-negative bacteria and therefore are useful pharmaceutical agents.

EP 0 132 501 A2

PATENTANWÄLTE
STREHL SCHÜBEL-HOPF SCHULZ
WIDENMAYERSTRASSE 17, D-8000 MÜNCHEN 22

DIPL ING. PETER STREHL
DIPL.-CHEM. DR URSULA SCHÜBEL-HOPF
DIPL.-PHYS. DR RÜTGER SCHULZ
AUCH RECHTSANWALT BEI DEN
LANDGERICHTEN MÜNCHEN I UND II

ALSO EUROPEAN PATENT ATTORNEYS

TELEFON (089) 223911
TELEX 5214036 SSSM D
TELECOPIER (089) 223915

0132501

EPA-13 863

28th March 1984

TITLE MODIFIED
see front page

IMIDAZOLE DICARBOXYLIC ACID DERIVATIVES, A PHARMACEUTICAL AGENT COMPRISING SAID IMIDAZOLE DICARBOXYLIC ACID DERIVATIVES AND A PROCESS FOR THEIR PRODUCTION

The present invention relates to novel mercapto derivatives of imidazole dicarboxylic acid and esters and salts thereof which are useful as antibacterial agents, particularly for the treatment of infectious diseases caused by gram-positive and gram-negative bacteria in human beings and animals. The present invention also relates to a process for their production as well as the use thereof.

In recent years, Pseudomonas aeruginosa, Klebsiella pneumoniae, Proteus spp., Serratia marcescens, etc. have become a problem as bacteria, which cause serious infectious diseases from the clinical aspect, and various drugs have been developed for treating these diseases.

For example, Japanese Patent Application 34795/78, U.S. Patent 4, 278,671 and British Patent 1 581 854 describe cephalosporin derivatives of the general

formula :

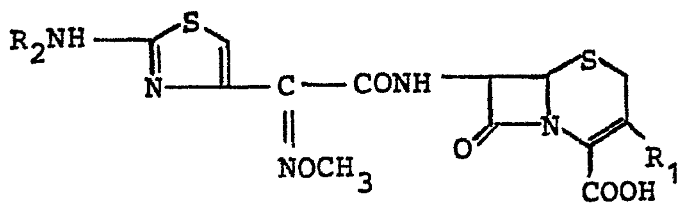

wherein $R_1$ is a group represented by $-CH_2R_3$ (wherein $R_3$ represents hydrogen or a residue of a nucleophilic compound) and $R_2NH-$ is an amino group which may be protected. In the stated Japanese Application compounds are specifically mentioned as concrete examples in which the 3-position of the cephalosporin derivatives is substituted by the residues of nucleophilic compounds, for example, a 1-methyl-1H-tetrazol-5-ylthiomethyl group. However, in practice these antibiotics are not satisfactory regarding their antibacterial activity against Pseudomonas aeruginosa.

Accordingly, it has been a strong need to develop novel antibiotics of the cephalosporin class showing a broad range of antibacterial activity, which are especially useful against Pseudomonas aeruginosa which has become the most serious problem.

The present inventors have found that compounds carrying a 4,5-dicarboxyimidazol-2-yl-thiomethyl group at the 3-position of the cephalosporin unit exhibit extremely excellent activity against gram-positive and gram-negative bacteria and also against Pseudomonas aeruginosa.

The present invention provides 2-mercapto-4,5-imidazole dicarboxylic acid derivatives represented by the following general formula (I) :

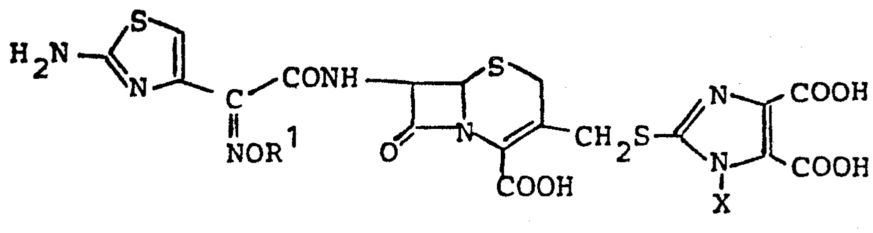

(I)

and esters and salts thereof, and it has been found that these compounds exhibit antibacterial activity against gram-positive and gram-negative bacteria over a wide spectrum, and exhibit potent antibacterial activity particularly against Pseudomonas aeruginosa. Their use as antibacterial agents or intermediates for producing antibacterial agents therefore is of high interest.

In the general formula (I) described above, $R^1$ represents a lower alkyl group having 1 to 4 carbon atoms such as a methyl group, an ethyl group, an isopropyl group, an n-butyl group, etc., X represents a hydrogen atom or a lower alkyl group having 1 to 4 carbon atoms which may have a substituent. Examples of the substituents include a carboxyl group, a dialkylamino group, etc.

The above mentioned esters are mono- and diesters of pharmaceutically acceptable alcohols, such as the tert-butyl ester, benzyl ester, silyl ester, trichloroethyl ester, diphenylmethyl ester.

Usable salts are pharmaceutically acceptable salts, examples of which include alkali metal salts, alkaline earth metal salts and salt of organic bases. The amino group may be in the free form or carry a protective group, for example, a benzyloxycarbonyl group, a tert-butyloxycarbonyl group, a chloroacetyl group, a trityl group or a formyl group.

The present invention further relates to the synthesis of the described novel compounds. For producing the compounds of the present invention for example, the following two processes can be adopted:

(1) Cephalosporins shown by general formula (II)

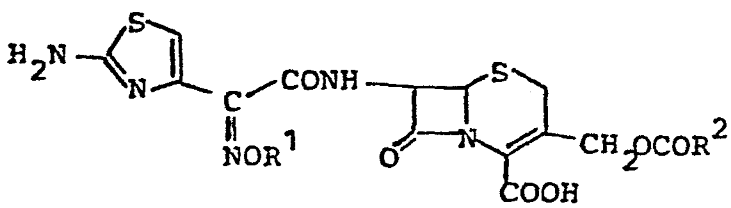

(II)

wherein $R^1$ and X are the same as defined hereinabove; $R^2$ represents a lower alkyl group such as a methyl group, an ethyl group, a butyl group, etc., or an aralkyl group such as a benzyl group, etc. are reacted with compounds of the general formula:

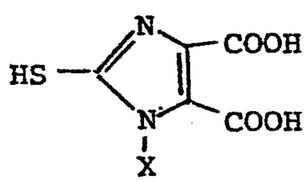

(IV)

or esters thereof, e. g. lower alkyl esters such as methyl, ethyl, n-butyl, etc., wherein X is the same as defined above, or aralkyl esters such as benzyl esters, etc.

The amino group may carry a protective group, as described above. When the product is an ester, it may be converted into the free form by e. g. an ester-removing reaction in a conventional manner.

In case the amino group carries a protective group, the protective group can be split off by conventional means for removing the protective group.

The reaction is carried out at a range from room temperature to 80 °C using an inert solvent, such as water, acetone, dioxan, acetonitrile, chloroform, methylene chloride, tetrahydrofuran (THF), dimethylformamide (DMF), formamide, etc. Hydrophilic organic solvents can also be used as a mixture with water. Further, if the reaction solvent is a mixture containing water, it is desired that the pH of the reaction liquid be controlled to 6 to 8 during the reaction.

(2) Cephalosporins shown by general formula (III) or esters thereof are subjected to a condensation-reaction with compounds shown by general formula (V) or reactive derivatives thereof:

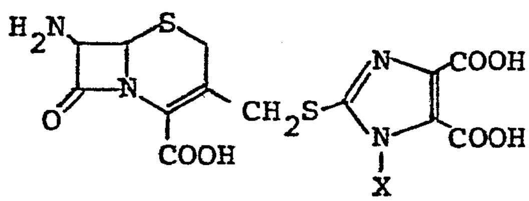

(III)

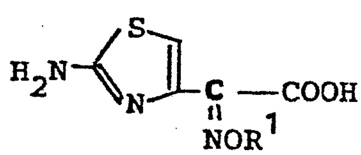

(V)

wherein $R^1$ and X are the same as defined hereinabove; and the amino group may be protected.

As described in (1), the carboxylic groups may be in the esterified form. The kind of the esters, the conversion thereof into the free form by an ester-removing reaction, the protection of the amino group and splitting off the protective group

are as defined above under (1).

As the condensation reaction, conventional condensation reactions are adopted.

Examples of suitable reactive derivatives of the compound (V) include acid halides, mixed acid anhydrides, active amides, active esters, etc. Particularly widely employed are acid chlorides, acid azides, dialkylphosphoric acid mixed anhydrides, phenylphosphoric acid mixed anhydrides, diphenylphosphoric acid mixed anhydrides, dibenzylphosphoric acid mixed anhydrides, halogenated phosphoric acid mixed anhydrides, dialkylphosphinic acid mixed anhydrides, sulfinic acid mixed anhydrides, thiosulfuric acid mixed anhydrides, sulfuric acid mixed anhydrides, alkylcarbonic acid mixed anhydrides, aliphatic carboxylic acid (e.g. pivalic acid, pentanoic acid, isopentanoic acid, 2-ethylbutanoic acid, trichloroacetic acid) mixed anhydrides, aromatic carboxylic acid (e. g. benzoic acid) mixed anhydrides, as well as symmetric acid anhydrides; acid amides with imidazole, 4-substituted imidazoles, dimethylpyrazole, triazole or tetrazole; cyanomethyl esters, methoxymethyl esters, vinyl esters, propargyl esters, p-nitrophenyl esters, 2,4-dinitrophenyl esters, trichlorophenyl esters, pentachlorophenyl esters, methanesulfonyl esters, phenylazophenyl esters, phenylthio-esters, p-nitrophenylthioesters, p-cresylthio-esters, carboxymethylthio-esters, pyranyl esters, pyridyl esters, piperidyl esters, 8-quinolylthio-esters; esters with N,N-dimethylhydroxylamine, 1-hydroxy-2-(1H)-pyridone, N-hydroxysuccinimide or N-hydroxyphthalimide, etc. Further,

in the condensation reaction, the $H_2N$-group in (V)

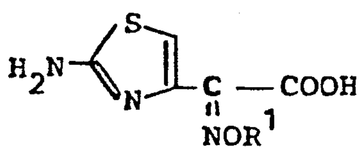

(V)

may be protected with protective groups conventionally used for protecting amino groups, such as a trityl group, a formyl group, a chloroacetyl group, etc.

The reaction of the aforesaid reactive derivatives with the compounds shown by general formula (III) is carried out in the presence of bases, such as alkali metal hydrogen carbonates, alkali metal carbonates, trialkyl amines, pyridine, etc. In the case of using solvents, these for example may be water, acetone, dioxan, acetonitrile, chloroform, methylene chloride, THF or DMF. Hydrophilic organic solvents can also be used in the form of a mixture with water. It is desired that the reaction be carried out generally under cooling or at room temperature.

Further, in the reaction with the reactive derivatives, the compounds shown by general formula (III) may be reacted in the form in which the carboxyl groups are converted into ester groups, such as t-butyl esters, benzyl esters, silyl esters, trichloroethyl esters, diphenylmethyl esters, etc. After completion of the reaction, these ester groups are split off in a conventional manner to obtain the free carboxyl groups.

The isolation of the reaction product may be carried out appropriately by conventional isolation methods such as extraction, column

chromatography, recrystallization etc. .

The thus obtained imidazole dicarboxylic acid derivatives can be converted into salts of inorganic bases such as alkali metal salts, e. g. sodium salts, potassium salts, etc.; alkaline earth metal salts such as calcium salts, barium salts, etc.; ammonium salts or salts of organic bases, such as lysine, procaine, pyrimidine, etc., in a conventional manner for preparing salts. These salts are preferred for producing medical preparations, because they are water soluble.

Additionally, if the derivatives of the present invention are used as antibiotics, they can be used in the form of derivatives which are converted into the aforesaid derivatives by the metabolic action in vivo, for example in the form of esters such as pivaloyloxymethyl esters.

In the use of the compounds for the therapeutic treatment there may be adopted for example the oral administration, intraperitoneal administration, subcutaneous administration, intravenous administration or intramuscular administration. In these cases, techniques for preparing medical preparations conventionally used for antibiotics are utilized. The dosage can be appropriately chosen depending upon the age and condition of the patient.

The invention further relates to a pharmaceutical, especially an antibiotic preparation containing the above-defined imidazole derivative as the active ingredient and which additionally may contain a usual pharmaceutical carrier and optionally usual pharmaceutical diluents, fillers, disintegrating agents and/or binders.

**Hereafter the present invention will be described in more detail with reference to the examples.**

Example 1

In 18 ml of a phosphate buffer having a pH of 6.8 were dissolved 1.43 g of 7-[2-methoxyimino-2-(2-amino-1,3-thiazol-4-yl)acetamido]cephalosporanic acid sodium salt (syn-isomer) and 0.62 g of 2-mercaptoimidazole-4,5-dicarboxylic acid. After adjusting the pH of the solution to 6.8 with 2N-NaOH, the solution was heated at 60 °C for 8 hours with stirring during which period the pH of the reaction solution was maintained at 6.8 by adding 2N-NaOH. After completion of the reaction, the reaction mixture was adsorbed to 700 ml of an adsorption resin "XAD-II" manufactured by Amberlite Co., Ltd. and eluted with water. Fractions containing the product were collected and freeze dried to obtain 0.8 g of the product, 7-[2-methoxyimino-2-(2-amino-1,3-thiazol-4-yl)acetamido]-3-(4,5-dicarboxyimidazol-2-yl)thiomethyl-3-cephem-4-carboxylic acid sodium salt (syn-isomer).

Infrared absorption spectrum (nujol): 1760 $cm^{-1}$ (ß-lactam)

Nuclear magnetic resonance spectrum ($D_2O$)

$\delta$ 3.55 (2H, q, 2-$CH_2$), 3.98 (3H, s =NO-$CH_3$), 4.08 (2H, q, 3-$CH_2$), 5.19 (1H, d, 6-H), 5.73 (1H, d, 7-H), 7.01 (1H, s, thiazole 5-H)

Mass spectrum (FAB-MS)

M + Na + H at M/Z 606 (1 Na salt, molecular weight, 605)

M + 2Na + H at M/Z 628 (2Na salt, molecular weight, 627)

M + 3Na + H at M/Z 650 (3Na salt, molecular weight, 649)

Example 2

By a similar manner as in Example 1, 0.88 g of 7-[2-methoxyimino-2-(2-amino-1,3-thiazol-4-yl)acetamido]-3-(1-methyl-4,5-dicarboxyimidazol-2-yl)thiomethyl-3-cephem-4-carboxylic acid sodium salt (syn-isomer) was obtained from 1.43 g of 7-[ 2-methoxyimino-2-(2-amino-1,3-thiazol-4-yl)acetamido]cephalosporanic acid sodium salt (syn-isomer) and 0.67 g of 1-methyl-2-mercaptoimidazole-4,5-dicarboxylic acid.

Infrared absorption spectrum (nujol): 1760 $cm^{-1}$ (β-lactam)

Nuclear magnetic resonance spectrum ($DMSO-d_6$)

δ 3.30 (2H, m, 2-$CH_2$), 3.80 (6H, b.s. imidazole 1-$CH_3$ and =NO-$CH_3$), 4.40 (2H, q, 3-$CH_2$), 4.94 (1H, d, 6-H), 5.52 (1H, m, 7-H), 6.70 (1H, s, thiazole 5-H), 7.20 (2H, b.s. $H_2N$-), 9.45 (1H, b.d. -CONH-)

Mass spectrum (FAB-MS)

| | |
|---|---|
| M + Na + H | at M/Z 620 (1Na salt, molecular weight 619) |
| M + 2Na + H | at M/Z 642 (2Na salt, molecular weight, 641) |
| M + 3Na + H | at M/Z 664 (3Na salt, molecular weight, 663) |

Example 3

To 18.9 g (0.1 mol) of iminodiacetic acid diethyl ester, 33 g (0.72 g) of formic acid was dropwise added with stirring. To the solution 30 g (0.3 mol) of anhydrous acetic acid was dropwise added with stirring. The mixture was gradually heated and further stirring and heating were conducted at 80°C for 1.5 hour. After concentration of the reaction mixture, the residue was distilled under reduced pressure. Components distilled off at 145 - 7°C/ 2 mm Hg were collected to obtain 18.5 g (yield 85%)

of N-formyliminodiacetic acid diethyl ester.

Nuclear magnetic resonance spectrum ($CDCl_3$)

$\delta$: 1.30 (6H, t, $-COOCH_2\underline{\underline{CH}}_3$), 4.15 (8H, m, $-N-\underline{\underline{CH}}_2-COO\underline{\underline{CH}}_2CH_3$); 8.10 (1H, s, HCO-)

To 2.15 g (95%, 0.03 mol) of sodium ethoxide there were added 20 ml of dry ether. With stirring, 4.4 g (0.03 mol) of diethyl oxalate was added to the mixture in small portions. After stirring for about 20 minutes, 6.5 g (0.03 mol) of N-formyliminodiacetic acid diethyl ester previously synthesized was dropwise added. Stirring was conducted at room temperature for 2.5 hours. After allowing to stand for 3 days, 25 ml of ice water was added to dissolve and the ether layer was separated. With ice-cooling and stirring 4.07 g (0.042 mol) of potassium thiocyanate and then 6 ml of 12N-HCl were added to the aqueous layer. To remove the remained ether, the mixture was somewhat concentrated and then heated at 50 to 60 °C for 7 hours with stirring.

After cooling in a refrigerator overnight, the precipitated solid was collected by filtration. After washing with 2N-HCl and then $H_2O$, the solid was dried under reduced pressure.

The obtained solid was added to a solvent mixture of ether and ethyl acetate (2 : 1) followed by stirring. After separating insoluble matters by filtration, the filtrate was concentrated to obtain 1.2 g of yellow orange powders of the product, 1-ethoxycarbonylmethyl-2-mercaptoimidazole-4,5-dicarboxylic acid diethyl ester, as the residue.

Nuclear magnetic resonance spectrum ($d_6$-DMSO)

$\delta$: 1.25 (9H, m, 3 kinds of $-COOCH_2\underline{\underline{CH}}_3$), 4.20 (6H, m, 3 kinds of $-COO\underline{\underline{CH}}_2CH_3$), 5.08 (2H, s, $-CH_2COO-$)

To 4.5 ml of 6N-NaOH was added 1.05 g (3.3 mmoles) of the thus obtained ester described above. The mixture was heated and stirred at 80°C for 1 hour. After treating with activated charcoal, 3.5 ml of conc. HCl was added under ice cooling. The aqueous solution was concentrated to about 10 ml and cooled to obtain 0.37 g of the product, 1-carboxymethyl-2-mercaptoimidazole-4,5-dicarboxylic acid, as yellow brown powders.

Nuclear magnetic resonance spectrum ($d_6$-DMSO)

$\delta$: 5.18 (2H, s, $-CH_2$-COO-)

By a similar manner as in Example 1, 0.344 g (1.4 mmol) of the thus obtained 1-carboxymethyl-2-mercaptoimidazole-4,5-dicarboxylic acid was allowed to react with 0.62 g (1.3 mmol) of 7- [2-methoxyimino-2-(2-amino-1,3-thiazol-4-yl)acetamido] cephalosporanic acid sodium salt (syn-isomer). After isolation and purification, 0.42 g of the product, 7-[2-methoxyimino-2-(2-amino-1,3-thiazol-4-yl)acetamido]-3-(1-carboxymethyl-4,5-dicarboxyimidazol-2-yl)thiomethyl-3-cephem-4-carboxylic acid sodium salt (syn-isomer) was obtained.

Infrared spectrum (nujol): 1760 $cm^{-1}$ (ß-lactam)

Nuclear magnetic resonance spectrum (DMSO-$d_6$)

$\delta$ 3.40 (2H, m, 2-$CH_2$), 3.82 (3H, s, =NO-$CH_3$), 4.32 (2H, q, 3-$CH_2$), 4.88 (2H, s, -$CH_2$COO at the 1-position of imidazole), 4.97 (1H, d, 6-H), 5.56 (1H, m, 7-H), 6.71 (1H, s, thiazole 5-H), 7.20 (2H, b.s. $H_2$N-), 9.48 (1H, b.d. -CONH-)

Mass spectrum (FAB-MS)

M + 2Na + H at M/Z 686 (2Na salt molecular weight, 685)

M + 3Na + H at M/Z 708 (3Na salt, molecular weight 707)

M + 4Na + H at M/Z 730 (4Na salt, molecular weight, 729)

Reference Example 1

In 10 ml of a phosphate buffer having pH 6.86 were dissolved 0.5 g of 7-[2-methoxyimino-2-(2-amino-1,3-thiazol-4-yl)acetamido]cephalosporanic acid sodium salt (syn-isomer) and 0.12 g of 2-mercaptoimidazole. After adjusting the pH of the solution to 6.8 with 2N-NaOH, the mixture was heated at 60°C for 8 hours with stirring, during which the pH of the reaction liquid was maintained at 6.8 with 2N-NaOH. After completion of the reaction, the reaction mixture was adsorbed to 250 ml of an adsorption resin "XAD-II", manufactured by Amberlite Co., Ltd. Fractions eluted out with 20 % methanol water which contained the product were collected. After freeze drying the fractions, 0.1 g of the product, 7-[2-methoxyimino-2-(2-amino-1,3-thiazol-4-yl)acetamido]-3-(imidazol-2-yl)thiomethyl-3-cephem-4-carboxylic acid sodium salt (syn-isomer) was obtained.

Infrared spectrum (KBr), 1760 $cm^{-1}$ (ß-lactam)

Nuclear magnetic resonance spectrum ($D_2O$)

$\delta$3.63 (2H, bs, 2-$CH_2$), 3.93 (3H, s, =N-$OCH_3$), 4.40 (2H, m, 3-$CH_2$), 5.16 (1H, d, J=6Hz, 6-H), 5.72 (1H, d, J=6Hz, 7-H), 6.90 (1H, s, thiazole 5-H), 7.33 (2H, s, imidazole 4,5-H)

Mass spectrum (FAB-MS)

M + H at M/Z 496 (free, molecular weight, 495)

M + Na + H at M/Z 518 (1Na salt, molecular weight 517)

The antibacterial activities of the compounds prepared by the above examples are shown in Table 1. From these results, it is understood that the compounds of the present invention have excellent

antibacterial activity as compared to the control compound and are useful as antibiotics. In particular, the compounds of the present invention are characterized by possessing carboxyl groups both at the 4- and 5-positions of the imidazole nucleus at the 3-position of cephalosporin. The synthesis of the compound without any carboxyl group is shown in Reference Example 1. As is noted from Table 1, it is understood that the antibacterial activity to Pseudomonas aeruginosa is greatly improved by the carboxyl groups. In addition, the compounds show a pronounced activity against Pseudomonas aeruginosa even compared with Cefotaxime or Cefmenoxime containing a $-OCOCH_3$ group or a group:

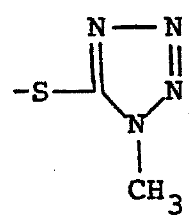

which is often employed conventionally as a substituent at the 3-position of cephalosporin. This activity is a significant characteristic of the compounds of the present invention.

Table 1. Antibacterial Activities of the Compounds of the Present Invention

| Example No. | 1 | 2 | 3 | Reference Example 1 | Cefmenoxime | Cefotaxime |
|---|---|---|---|---|---|---|
| Pseudomonas aeruginosa IFO 3445 | 3.13 | 6.25 | 6.25 | 25 | 25 | 12.5 |
| Pseudomonas aeruginosa 5 | 1.56 | 3.13 | 6.25 | 25 | 12.5 | 12.5 |
| Escherichia coli | 0.10≧ | 0.10≧ | 1.56 | 0.10≧ | 0.10≧ | 0.10≧ |
| Klebsiella pneumoniae | 0.10≧ | 0.10≧ | 0.20 | 0.10≧ | 0.10≧ | 0.10≧ |
| Proteus vulgaris | 0.10≧ | 0.10≧ | 0.10= | 0.10≧ | 0.10≧ | 0.10≧ |
| Proteus mirabilis | 0.10≧ | 0.10≧ | 0.10= | 0.20 | 0.39 | 0.10≧ |
| Serratia marcescens | 0.20 | 0.20 | 0.78 | 0.78 | 0.78 | 0.20 |

The values of MIC were measured by the agar dilution method.

Cefmenoxime

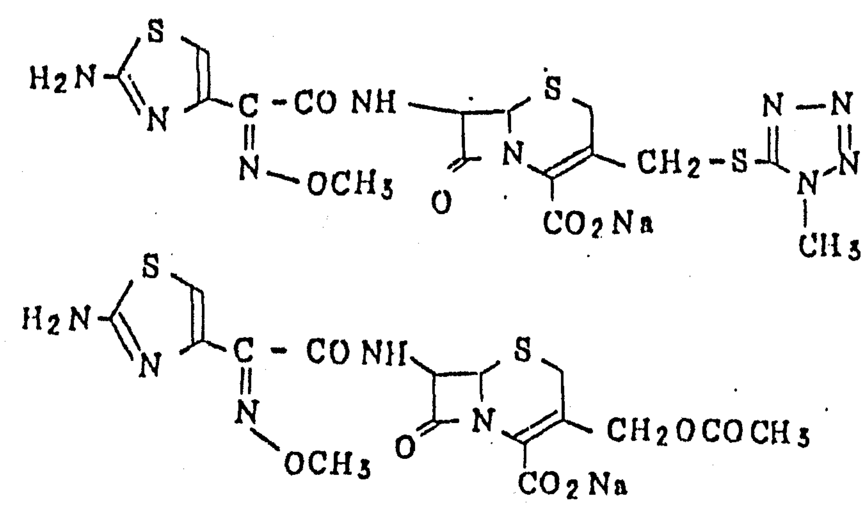

Cefotaxime

CLAIMS

1. An imidazole dicarboxylic acid derivative of the general formula (I)

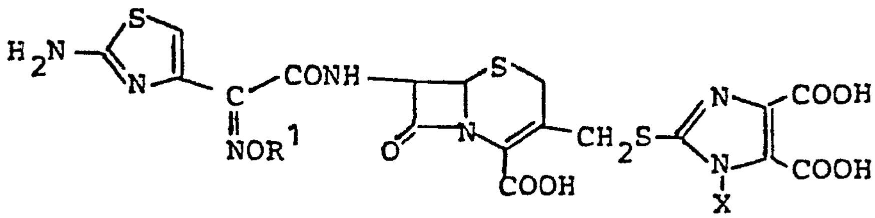

(I)

wherein $R^1$ represents a lower alkyl group and X represents a hydrogen atom or a lower alkyl group which may have a substituent, and in which the amino group may carry a protective group, and esters and salts thereof.

2. A pharmaceutical agent for treating and preventing infectious diseases, characterized in that it contains or consists of at least one imidazole dicarboxylic acid derivative of the general formula (I) according to claim 1, a pharmaceutically acceptable ester or salt thereof or a compound which in vivo is converted into such derivative or its salt or ester.

3. A process for the production of an imidazole dicarboxylic acid derivative of the general formula (I) according to claim 1, which comprises reacting a compound of the general formula:

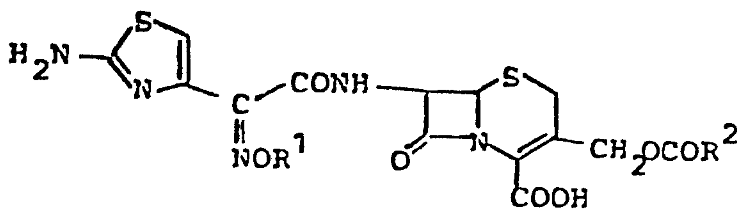

(II)

with a compound of the general formula

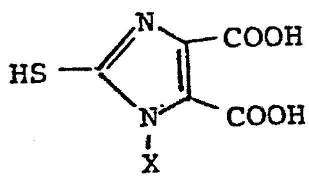

(IV)

or an ester thereof and, when the product is an ester, subjecting it to a further reaction for removing the ester group; wherein $R^1$ represents a lower alkyl group, $R^2$ represents a lower alkyl group or an aralkyl group; X represents a hydrogen atom or a lower alkyl group which may have a substituent, and in which the amino group may carry a protective group.

4. A process for the production of an imidazole dicarboxylic acid derivative of the general formula (I) according to claim 1, which comprises reacting a compound of the general formula:

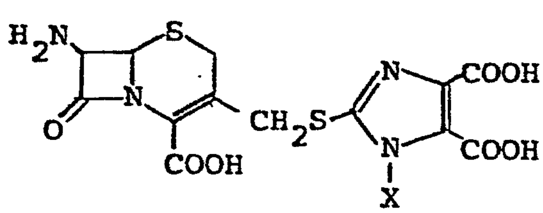

(III)

or an ester thereof with a compound of the general formula

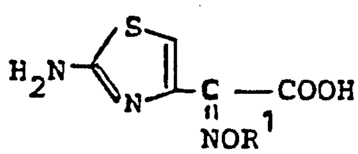

(V)

or a reactive derivative thereof and, when the product is an ester, subjecting it to a further reaction for removing the ester group, wherein $R^1$ represents a lower alkyl group and X represents a hydrogen atom or a lower alkyl group which may have a substituent, and in which the amino group may carry a protective group.